# EUROPEAN PATENT APPLICATION

(11) **EP 1 330 995 A2**
(43) Date of publication of application: **30.07.2003**
(21) Application number: 03250346.8
(22) Date of filing: 21.01.2003
(51) Int. Cl.: A61F 13/533

(54) **Absorbent body for hygienic absorbent articles**

(30) Priority: 23.01.2002 JP 2002014892; 15.11.2002 JP 2002335122
(71) Applicant: Uni-Charm Corporation, Kawanoe-shi, Ehime-ken 182 (JP)
(72) Inventor: Nanaumi, Hisataka, Technical Center, Mitoyo-gun, Kagawa-ken 769-1602 (JP); Kashiwagi, Masahiro, Technical Center, Mitoyo-gun, Kagawa-ken 769-1602 (JP); Komatsu, Shinpe, Technical Center, Mitoyo-gun, Kagawa-ken 769-1602 (JP)
(74) Representative: Parry, Christopher Stephen

(57) **Abstract**

Disclosed is an absorbent body including: an absorbent core including cellulose fibers; and hydrophilic paper covering a body surface and a garment surface of the absorbent core. The absorbent body is embossed in a predetermined dot pattern to have a plurality of recesses in which the absorbent core and the paper are compressed and integrated together.

## Description

### BACKGROUND OF THE INVENTION

### Field of the Invention

The present invention relates to an absorbent body suitable for use in an absorbent article such as a sanitary napkin, an incontinence pad for light incontinence discharges or the like. The present invention also relates to an absorbent article using the absorbent body.

### Description of the Related Art

Absorbent bodies of the type having an absorbent core that has fluff pulp as main component (the fluff pulp is sometimes mixed with superabsorbent resin for increasing absorbency) wrapped in tissue paper have been widely used for absorbent articles. In such an absorbent article, the absorbent body is generally disposed between a liquid permeable topsheet and liquid impermeable backsheet that are wider than the absorbent body, and the topsheet and the backsheet are bonded together outside the absorbent body. Along its peripheral edge, the absorbent article is sealed so as to prevent leakage.

In such a conventional absorbent body, the tissue paper functions as a sheet for collecting absorbent materials such as fluff pulp during the production process. After absorbent materials are deposited thereon, side portions of the tissue paper outside the deposited absorbent materials are folded back to overlap with each other, so that the tissue paper functions as a wrapping sheet for stabilizing the shape of the absorbent body. When used in the absorbent article, moreover, a portion of the tissue paper beneath the liquid permeable topsheet functions as a diffusion layer for body fluids given to the liquid permeable topsheet, so that a large area of the absorbent body can be utilized for efficiently absorbing body fluids.

Upon use, an absorbent article such as a sanitary napkin is fixed to an undergarment such as a short panty through a pressure sensitive adhesive and then applied to the private parts of a wearer. At this time, even if the absorbent article is subjected to various forces such as a shearing force due to motion of the wearer's body, initial positional relationship between the individual components of the absorbent article need be maintained so as to exhibit the function. That is, if the topsheet should be easily separated from the tissue paper (absorbent body) due to motion of the wearer's body or the absorbent body (absorbent core) should be twisted or shifted to leave a space between the absorbent body and the topsheet or the backsheet, an initially intended absorbency necessary for the absorbent article cannot be exhibited, causing lateral leakage or the like.

Accordingly, the individual components of the absorbent article are bonded to each other through a hot-melt adhesive or by thermal-bonding so as not to separate from each other. Japanese Unexamined Patent Publication No. H9-168563 (168563/1997) discloses an absorbent article having an absorbent body between a topsheet and a backsheet, of which the topsheet and the absorbent body are heat-embossed to have longitudinally extending linear embossments at left and right side portions of the absorbent article.

In the case where the individual components of the absorbent article are bonded to each other through a hot-melt adhesive as set forth above, it may be possible to appropriately bond the absorbent body (i.e., the absorbent core wrapped in the tissue paper) to the topsheet and the backsheet. However, it is not easy to join the tissue paper to the absorbent core so as not to separate from each other. Therefore, the tissue paper may adhere to the discharging part of a wearer together with the topsheet, while separating from the absorbent core. In this case, since body fluids given to the topsheet cannot readily migrate to the absorbent core, they may possibly leak sideways along the topsheet.

Also in the case where the absorbent article is provided at its left and right side portions with embossments where the topsheet and the absorbent body are recessed, as disclosed in the above-identified Patent Publication, the tissue paper is liable to separate from the absorbent core in a region between the left and right embossments.

### SUMMARY OF THE INVENTION

The present invention has an object to provide an absorbent body having an absorbent core of cellulose fibers such as fluff pulp wrapped in hydrophilic paper such as tissue paper, wherein the absorbent core and the paper are certainly joined together without excessively increasing a stiffness of the entire absorbent body. It is another object of the present invention to provide an absorbent article using the absorbent body.

According to a first aspect of the present invention, there is provided an absorbent body comprising:
an absorbent core including cellulose fibers; and
hydrophilic paper covering a body surface and a garment surface of the absorbent core, wherein
the absorbent body is embossed in a predetermined dot pattern to have a plurality of recesses in which the absorbent core and the paper are compressed and integrated together.

As a result of various considerations for solving the above-mentioned'shortcoming, the present inventors have found that even if neither the hydrophilic paper (e.g., tissue paper) nor the absorbent core forming the absorbent body does not contain heat-fusible fibers, they can be firmly joined together without increasing the stiffness of the entire absorbent body to an undesirable extent by deeply embossing them in a predetermined dot pattern to distribute a plurality of recesses . Accordingly, the present invention has been accomplished.

In the present invention, the paper may be broken in at least some of the recesses.

In one preferred embodiment of the present invention, the recesses may include first recesses and second recesses having a smaller area than the first recesses, and both the first and second recesses may be dotted over the absorbent body. In this case, it is preferred that the first and second recesses are arranged such that each first recess is surrounded by a plurality of second recesses. It is also preferred that the paper is broken in at least some of the second recesses.

If the paper is broken in the recesses due to the pressure of embossing, fibers of the paper at the broken portions can moderately entangle with fibers of the absorbent core to further increase the joining strength between the paper and the absorbent core at the recesses.

If the recesses include the first and second recesses that are different in size and the recesses are dotted such that each first recess is surrounded by a plurality of smaller second recesses, as set forth above, easy separation of the paper from the absorbent core can be prevented by increasing the number of the recesses, while excessive increase of the stiffness of the absorbent body due to the presence of the recesses can be prevented since the first recesses can be made small.

Preferably, each recess has a maximum length of 0.5 to 4 mm at a bottom thereof, and a center-to-center distance between adjacent recesses is 2 to 8 mm.

The absorbent body may be compressed not only at the recesses but also as a whole.

Preferably, the absorbent body has a Gurley stiffness of 2.45 to 3.43 mN.

According to a second aspect of the present invention, there is provided an absorbent article having the absorbent body disposed between a backsheet and a liquid permeable topsheet, wherein the paper covering the body surface and the garment surface of the absorbent core is bonded to both a member laid on a body surface of the absorbent body and a member laid on a garment surface of the absorbent body.

In the absorbent article of the present invention, since the absorbent body is bonded to both the topsheet and the backsheet, and the absorbent core and the paper of the absorbent body are integrated together by embossing, the topsheet hardly separates from the paper and the absorbent core. Therefore, body fluids having been given to and passed through the topsheet can be certainly diffused through the paper and then absorbed by the absorbent core.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
Fig. 1 is a top plan view showing an absorbent body according to one embodiment of the present invention, as viewed from the side of a body surface;
Fig. 2 is a top plan view showing a portion of Fig. 1 on larger scale for better understanding of a dot-embossed pattern of Fig. 1;
Figs. 3A and 3B are sectional views showing recesses of the absorbent body;
Fig. 4 is a top plan view showing a sanitary napkin using the absorbent body of Fig. 1, as viewed from the side of a body surface;
Fig. 5 is a sectional view of the sanitary napkin of Fig. 4 taken along line V-V coinciding with a transversely extending centerline Ox-Ox;
Fig. 6 is a top plan view showing a reinforcing member used in the sanitary napkin; and
Fig. 7 is an enlarged cross-sectional view showing a portion of the reinforcing member of Fig. 6, wherein the reinforcing member is cut in thickness direction.

### DESCRIPTION OF THE PREFERRED EMBODIMENT

The present invention will be discussed hereinafter in detail in terms of the preferred embodiment according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

As used herein, the term " absorbent article" means an article that is intended to be worn in the crotch of a wearer's body for absorbing discharged body fluids such as menstrual blood, urine and vaginal discharge. In the following embodiment, the absorbent article of the present invention will be described embodied in a sanitary napkin that is mainly intended to absorb menstrual blood discharged from the vaginal opening of a woman. It should be noted that the absorbent article and components thereof have two surfaces, of which one surface that is intended to be directed toward the crotch of a wearer is called " body surface," while the other surface that is on the opposite side is called " garment surface" regardless of whether or not an garment is worn over the absorbent article.

As used herein, the term " transversely extending centerline" means a line extending in a transverse direction of the absorbent article to divide a length of the absorbent article into two equal parts, while the term " longitudinally extending centerline" means a line extending in a longitudinal direction of the absorbent article to divide a width of the absorbent article into two equal parts.

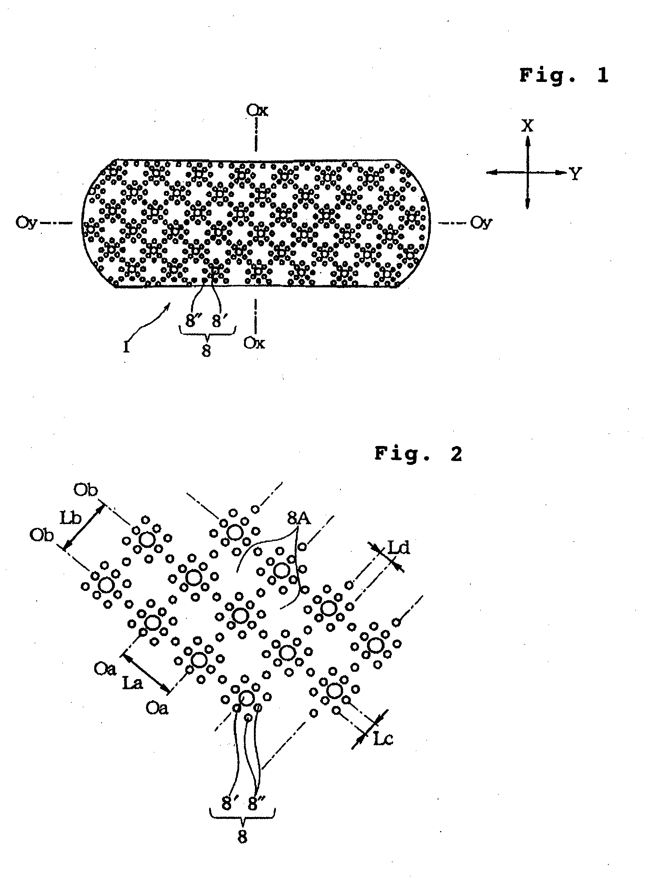

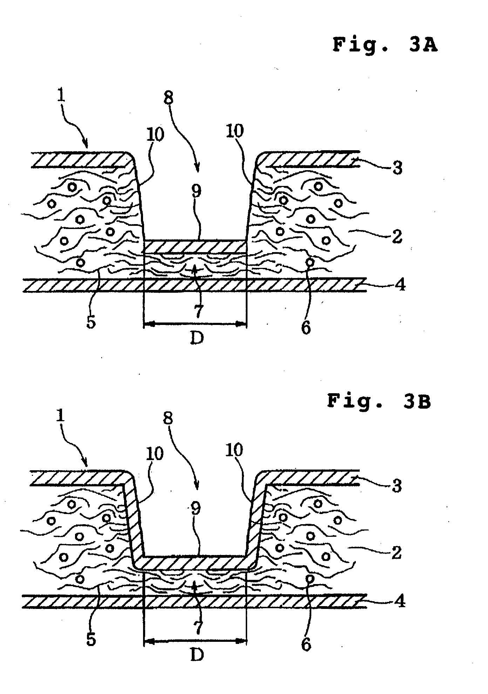

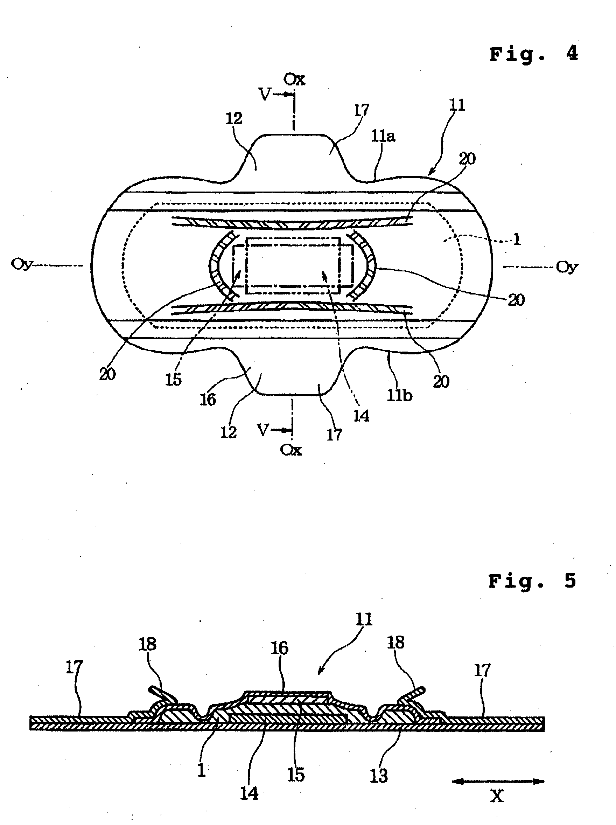

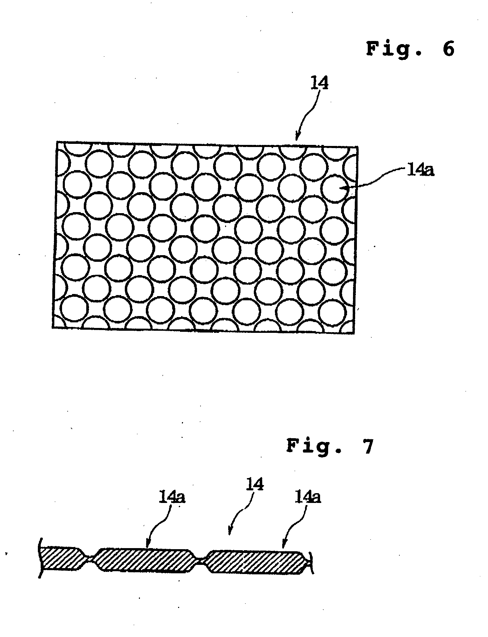

Fig. 1 is a top plan view showing an absorbent body according to one embodiment of the present invention, as viewed from the side of the body surface thereof; Fig. 2 is a top plan view showing a portion of Fig. 1 on larger scale for better understanding of a dot-embossed pattern of Fig. 1; Figs. 3A and 3B are sectional views showing a portion of the absorbent body on larger scale where recesses are formed by embossing; Fig. 4 is a top plan view showing a sanitary napkin using the absorbent body of Fig. 1, as viewed from the side of the body surface thereof; and Fig. 5 is a sectional view of the sanitary napkin taken along line V-V coinciding with a transversely extending centerline Ox-Ox.

Referring to Figs 1 to 5, the absorbent body is indicated by 1. The absorbent body 1 comprises: an absorbent core 2; and tissue paper layers 3 and 4 (hydrophilic paper) covering the body surface and the garment surface of the absorbent core 2, respectively.

The absorbent core 2 is mainly comprised of fluff pulp (comminuted pulp) 5 that is natural cellulose fibers. Preferably, the absorbent core 2 is formed by blending the fluff pulp 5 with superabsorbent resin 6 such as polyacrylate polymer. The basis weight of the fluff pulp 5 is in the range of 150 to 210 g/m². In the case where the fluff pulp 5 is blended with the superabsorbent resin 6, the superabsorbent resin 6 is distributed within the range of 10 to 30 g/m² uniformly inside the absorbent core 2 or in layers inside the absorbent core 2 in the thickness direction thereof. Cellulose fibers for use in the absorbent core 2 should not be limited to the fluff pulp 5. For example, the fluff pulp 5 may be blended with regenerated cellulose fibers such as rayon. It is also possible to add chemical pulp.

For the tissue paper layers 3 and 4, used is paper having a basis weight of 10 to 40 g/m², which is prepared by forming a sheet from ordinary pulp and optionally adding a binder. The tissue paper layer 3 covering the body surface of the absorbent core 2 and the tissue paper layer 4 covering the garment surface of the absorbent core 2 may be of the same basis weight or different basis weights.

The absorbent body 1 may be prepared as follows. While a band-shaped tissue paper is fed, comminuted pulp or a blend of comminuted pulp and superabsorbent resin is supplied from a hopper or the like and collected on a central portion of the tissue paper in a width of about one half the width of the tissue paper (the width of the tissue paper means a size in a direction perpendicular to the feed direction of the tissue paper) to have a predetermined basis weight and a predetermined shape. Thus, the absorbent core 2 can be formed on the central portion of the tissue paper to extend in the feed direction. After forming the absorbent core 2 on the tissue paper, transversely opposed side portions of the tissue paper are folded back to cover the upper surface of the absorbent core 2, and then, the absorbent core 2 wrapped in the tissue paper is shaped through a compression roll. Thereafter, not only the tissue paper but also the absorbent core 2 wrapped in the tissue paper is cut into a predetermined shape and size, producing the absorbent body 1. In this production process of the absorbent body 1, the tissue paper preferably has such a width that the transversely opposed side portions of the tissue paper folded back after formation of the absorbent core 2 can overlap with each other. Alternatively, the tissue paper may be so narrow that the folded-back side portions cannot overlap with each other. In this case, after formation of the absorbent core 2 on the tissue paper, another tissue paper may be put on the absorbent core 2 to cover the whole surface of the absorbent core 2. It also should be noted that when the tissue paper and the absorbent core 2 wrapped in the tissue paper are cut after compression with the compression roll, longitudinally extending side edges may or may not be cut off as long as the whole body surface and the whole garment surface of the absorbent core are covered with the tissue paper.

The absorbent body 1 thus produced has a thickness of 1 to 3 mm.

The absorbent body 1 is embossed from the tissue paper layer 3 toward the tissue paper layer 4 with substantially cylindrical pins (not shown) heated to about 160 to 180 °C, so that recesses 8 having a substantially circular top plan shape are dotted over the absorbent body 1. Referring to Figs. 3A and 3B, the bottom of each recess 8 is indicated by 9.

As shown in Figs. 3A and 3B, the absorbent core 2 is compressed more beneath the bottoms 9 of the recesses 8 than in the other portions. In the absorbent body 1, the portions beneath the bottoms 9 that are compressed to have a higher density than the other portions and that can maintain such a compressed state are called consolidated portions 7. As shown in Fig. 1, the recesses 8 are dotted over the surface of the absorbent body 1 in a predetermined pattern, and the bottom 9 of the recess 8 is of a substantially circular shape. However, the bottom 9 of the recess 8 may be of an elliptical shape, an oval shape, a quadrangular shape or any other shape.

In the consolidated portions 7, the tissue paper layer 3, the absorbent core 2 and the tissue paper layer 4 are compressed and integrated together with the fluff pulp 5 forming the absorbent core 2 and the pulp forming the tissue paper layers 3 and 4 being hydrogen bonded to one another due to the hydroxyl group on the surface thereof. Such hydrogen bonding can be accomplished by embossing the absorbent body 1 with the heated embossing pins under a predetermined humidity.

In the embodiment shown, as will be described in detail later, the recesses 8 include first recesses 8' having a large area and second recesses 8" having a smaller area than the first recesses 8' . In both the first and second recesses 8' and 8" , the circular bottom 9 preferably has a diameter D of 0.5 to 4 mm, which is almost equal to a diameter of the consolidated portion 7. In the case where the bottom 9 is of an elliptical shape, an oval shape, a quadrangular shape or any other shape, on the other hand, the bottom 9 preferably has a maximum length of 0.5 to 4 mm. It is also preferred that a center-to-center distance between adjacent recesses is in the range of 2 to 8 mm, wherein the term " adjacent recesses" refers to an arbitrary recess and the nearest one of the other recesses.

In the embodiment of Fig. 1, the recesses 8 are dotted over the whole body surface of the absorbent body 1 (the whole tissue paper layer 3). However, the recesses 8 may be dotted exclusively along a peripheral edge of the absorbent body 1, longitudinally opposed end edges of the absorbent body 1, or transversely opposed side edges of the absorbent body 1. In other words, a region having no recesses 8 may be provided centrally of the absorbent body 1, along the transversely extending centerline Ox-Ox of the absorbent body 1, or along the longitudinally extending centerline Oy-Oy of the absorbent body 1.

Since the substantially circular recesses 8 are deeply formed by embossing the absorbent body 1 from the side of the tissue paper layer 3, as shown in Figs. 3A and 3B, the tissue paper layer 3, the compressed absorbent core 2 and the tissue paper layer 4 are tightly integrated in the consolidated portions 7 beneath the bottoms 9 of the recesses 8.

Each recess 8 has an inner wall 10 that rises at an angle or vertically from the periphery of the bottom 9, as shown in Fig. 3. When the tissue paper layer 3 is stretched by embossing, it is preferred that some of the fibers forming the tissue paper layer 3 are cut to cause breakage of the tissue paper layer 3 at the inner wall 10 and forced into the fluff pulp 5 of the absorbent core 2 inside the tissue paper layer 3 to cause entanglement with the fluff pulp 5 of the absorbent core 2. In this case, even if both the tissue paper layer 3 and the absorbent core 2 are formed of cellulose fibers without having heat-fusible fibers, the tissue paper layer 3 and the absorbent core 2 can be firmly joined together by formation of the recesses 8.

However, even if the tissue paper layer 3 is not broken at the inner wall 10 of the recess 8 (i.e., the constituent fibers of the tissue paper layer 3 and the fluff pulp 5 of the absorbent core 2 are not entangled with each other unlike Fig. 3A) as shown in Fig. 3B, since the constituent fibers of the tissue paper layer 3, the fluff pulp 5 of the absorbent core 2 and the constituent fibers of the tissue paper layer 4 are highly compressed under heat in the consolidated portions 7 to form hydrogen bonds and also given a pressure, there can be obtained the absorbent body 1 wherein the tissue paper layer 3 and the tissue paper layer 4 are hardly separated from the absorbent core 2.

Here, it is preferred that the tissue paper layer 3 (more preferably both the tissue paper layer 3 and the tissue paper layer 4) is broken in some of the recesses 8, for example, in at least 3% (more preferably at least 5%) of the total number of the recesses 8, as shown in Fig. 3A. It this case, of course, the tissue paper is not broken in the remaining recesses 8, as shown in Fig. 3B. However, it is more preferred that the tissue paper is broken in most (e.g., at least 50% of the total number) of the recesses 8.

Since the recess 8 is of a substantially circular shape as viewed from above, the boundary between the recess 8 and the not-recessed surface of the absorbent body 1 is also substantially circular. Therefore, peeling force or shearing force acting between the tissue paper layer 3 and the absorbent core 2 can be distributed along the boundary without concentrating at one point of the boundary, so that the problem of separation of the tissue paper layer 3 from the absorbent core 2 can be prevented.

Moreover, since the recesses 8 are dotted in spaced relation to each other, peeling force or shearing force acting between the tissue paper layer 3 and the absorbent core 2 can be distributed to several recesses 8 without being concentrated at only one recess 8, so that the tissue paper layer 3 can be prevented from easily separating from the absorbent core 2.

Since the tissue paper layer 3 and the absorbent core 2 are firmly joined to each other at the inner wall 10 and the bottom 9 of each recess 8 as described hereinabove, the absorbent body 1 can easily withstand peeling force or shearing force acting between the tissue paper layer 3 and the absorbent core 2. In addition, since the peeling force or shearing force can be distributed as described hereinabove, the problem of separation of the tissue paper layer 3 from the absorbent core 2 can be prevented. Since the tissue paper layer 3 can keep in contact with the absorbent core 2 during use of an absorbent article, body fluids having migrated from a topsheet of the absorbent article to the tissue paper layer 3 and dispersed therein can be stably introduced into the absorbent core 2 that is in close contact with the tissue paper layer 3.

In order that the absorbent body can exhibit such an initially intended function, however, the stiffness of the absorbent body 1 should not be excessively increased due to the presence of the consolidated portions 7. This is because if the stiffness is excessively increased, the absorbent article cannot easily follow the motion of the wearer's body and a fit against the private parts will be deteriorated during use. The stiffness of the absorbent body 1 is preferably equal to or less than 3.92 mN (400 mgf) in accordance with the Gurley Method. More preferably, the Gurley stiffness is in the range of 2.45 to 3.43 mN (250 to 350 mgf). As used herein, the Gurley stiffness is expressed by a value measured by using a Gurley stiffness tester manufactured by Yasudaseiki Sei-sakusho, Ltd., wherein the absorbent body 1 is cut into a size having a length of 51 mm in Y-direction and a length of 12.7 mm in X-direction.

Here, the diameter D of the bottom 9 of the substantially circular recess 8 and the ratio of the area occupied by recesses 8 to the surface of the absorbent body 1 having the recesses 8 should be taken into consideration. If the diameter D is too large, the stiffness is locally increased to affect the flexibility of the entire absorbent body 1. If the ratio of the area occupied by the recesses 8 is too large, the stiffness of the entire absorbent body 1 is increased to deteriorate a fit against the private parts.

From this view point, the diameter D of the bottom 9 of the substantially circular recess 8 is preferably in the range of 0.5 to 4 mm, as described hereinabove, and the ratio of the area occupied by the recesses 8 to the surface of the absorbent body 1 is preferably equal to or less than 15%, more preferably equal to or less than 10%.

Considering both the peel strength of the tissue paper layers 3, 4 and the stiffness of the absorbent body 1, it is preferred that the recesses 8 are uniformly dotted over the surface of the absorbent body 1, and it is more preferred that the recesses 8 includes two or more kinds of recesses having different diameters (maximum lengths). This can result in that the joining strength can be increased by increasing the number of joining between the absorbent core 2 and the tissue paper layers 3, 4 and that the stiffness of the entire absorbent body 1 can be easily adjusted so as not to excessively increase.

In the embodiment shown in Figs. 1 and 2, for example, the recesses 8 are dotted over the surface of the absorbent body 1 in such a regular pattern that a small number of first circular recesses 8' having a large diameter (maximum length) at the bottom 9 thereof and a large number of second circular recesses 8" having a smaller diameter (maximum length) at the bottom 9 thereof than the first recesses 8'. More specifically, the recesses 8 are arranged in such a pattern that the first recesses 8' having a larger diameter are dotted over the surface of the absorbent body 1 and the second recesses 8" having a smaller diameter are dotted to surround the individual first recesses 8'. Accordingly, the joining strength between the tissue paper layers 3, 4 and the absorbent core 2 can be increased by the first recesses 8' and the second recesses 8" concentrated to surround the individual first recesses 8', while the stiffness of the absorbent body 1 can be prevented from being locally extremely increased because adjacent first recesses 8' having a larger diameter can be spaced sufficiently apart from each other, so that the absorbent body 1 can easily deform in accordance with the motion of the wearer's body.

Figs. 1 and 2 illustrate one example of the arrangement pattern of the recesses. In this case, the first recesses 8' having a diameter of 2 to 4 mm at the bottom 9 thereof are located on intersections of reference lines Oa and Ob that are inclined at an angle of 30 to 60 degrees (at 45 degrees in the drawings) to both the longitudinal direction (Y-direction) and the transverse direction (X-direction) and perpendicularly intersect each other. Both the pitch La of the reference lines Oa and the pitch Lb of the reference lines Ob are in the range of 13 to 23 mm. On the other hand, the second recesses 8" having a diameter of 0.5 to 1.5 mm at the bottom 9 thereof are arranged at a constant pitch on the reference lines Oa and Ob, and are also arranged in a square pattern to surround the individual first recesses 8'. The center-to-center distances Lc and Ld between adjacent second recesses 8" are in the range of 2 to 8 mm. The center-to-center distance between adjacent first and second recesses 8' and 8" is also in the range of 2 to 8 mm.

In a region surrounded by the reference lines Oa and Ob, nonembossed regions 8A having no recesses are left. The center-to-center distance between second recesses 8" at opposite sides of each nonembossed region 8A is at least twice the distance Lc and Ld, so that the nonembossed region 8A is a region where the absorbent core 2 is not substantially compressed.

In the shown embodiment, the total area of the recesses 8, i.e., the sum of the area of the first recesses 8' and the area of the second recesses 8" is 6% of the surface area of the absorbent body 1.

For example, when an absorbent body 1 having a basis weight of 170 g/m² was prepared and heat-embossed in a pattern of Figs. 1 and 2, the density of the consolidated portion 7 at the bottom 9 of the first recess 8' was 0.82 g/cm³, and the density of the consolidated portion 7 at the bottom 9 of the second recess 8" was 1.12 g/cm³.

Here, heat-embossing for forming the substantially circular recesses 8 may be performed on the absorbent body 1 immediately after the absorbent core 2 is wrapped in the tissue paper. Alternatively, before heat-embossing the absorbent body 1 to form the substantially circular recesses 8, the absorbent body 1 may be made thin by compressing (e.g., heat-embossing or heat-pressing) the absorbent body 1 between pressing rolls. Needless to say, heat-embossing or heat-pressing for making the absorbent body 1 thin is not performed under a pressure as high as in case of heat-embossing for forming the substantially circular recesses 8, but performed under a pressure as low as can make the entire absorbent body 1 thin to some extent.

Such compression for making the absorbent body thin has an advantage in that a sanitary napkin or the like using the absorbent body can be comfortably worn and also has an effect of preventing unstable state such as easy disentanglement of the fluff pulp of the absorbent core 2 due to shearing force or the like.

For example, a compressed absorbent heat-embossed under such a low pressure was prepared. This absorbent body had a peel strength of 931 mN/25mm between the tissue paper layers 3, 4 and the absorbent core 2. Even after the tissue paper layer 3 was peeled from the absorbent core 2, breakage (tear) was not found in the tissue paper layer 3 at a location where embossing was performed under low pressure. Next, the compressed absorbent body heat-embossed under low pressure was further embossed from the side of the body surface to form the circular recesses 8 having a diameter of 1 mm and the consolidated portions 7 beneath them. This absorbent body had a peel strength of 1960 mN/25mm, and after the tissue paper layer 3 was peeled from the absorbent core 2, it was confirmed that structures of the tissue paper layer 3 and the absorbent core 2 were destroyed at the inner walls 10 of the recesses 8, so that the constituent fibers of the tissue paper layer 3 and the fluff pulp of the absorbent core 2 were entangled about each other.

The peel strength as used herein means a maximum force that is measured when the tissue paper layer 3 is pulled in the 180° direction at a rate of 100 mm/minute, wherein the absorbent body 1 has a width of 25 mm in the transverse direction (X-direction) and a length of 100 mm in the longitudinal direction (Y-direction). In order to effectively prevent peeling of the tissue paper layer 3 from the absorbent core 2 during wear, the peel strength is preferably equal to or greater than 1500 mN/25mm.

It should be noted that heat-embossing has been described hereinabove to be performed from the side of the tissue paper layer 3, but may also be performed from the side of the tissue paper layer 4. Alternatively, heat-embossing may be performed from both the side of the tissue paper layer 3 and the side of the tissue paper layer 4.

The basic shape of the absorbent body 1 wherein the absorbent core 2 is wrapped in the tissue paper is usually rectangle, but it is needless to say that absorbent bodies that are variously changed in shape (e.g., curved) so as to improve function or design thereof can be encompassed within the scope of the invention as long as the technical idea of the present invention is used.

Figs. 4 and 5 show a sanitary napkin 11 that is elongated in the Y-direction as viewed from above. This sanitary napkin 11 has wing portions 12, 12 projecting from the opposite side ends 11a, 11b that are equally spaced apart from the longitudinally extending centerline Oy-Oy.

In the sanitary napkin 11, as shown in Figs. 4 and 5, a liquid impermeable backsheet 13 is provided to appear on the garment surface of the napkin 11, and the absorbent body 1 is provided on the backsheet 13 with a reinforcing member 14 disposed therebetween. On the absorbent body 1, there is provided a liquid guide layer 15. A liquid permeable topsheet 16 is provided to appear on the body surface of the napkin 11. The topsheet 16 may be a nonwoven fabric, a nonwoven fabric processed to have liquid passage holes, a resin film processed to have liquid passage holes, or the like. In the preferred embodiment, the topsheet 16 is of two-layer structure having: a resin film processed to have a large number of liquid passage holes for appearing on the body surface of the napkin 11; and an air-laid nonwoven fabric laid on the garment surface of the resin film. On the other hand, the backsheet 13 is a liquid impermeable resin film.

Furthermore, the sanitary napkin 11 is provided at left and right side portions with liquid impermeable side sheets 17, 17 that appear on the body surface of the napkin 11, as shown in Figs. 4 and 5. The individual side sheet 17 is extended transversely inwardly to cover the backsheet 13 in the wing portion 12 and overlap with the topsheet 16 along its side edge extending along the longitudinally extending centerline Oy-Oy, and then folded back transversely outwardly. The folded-back portion of the side sheet 17 is raised from the topsheet 16 to thereby form a leakage preventing wall 18.

As shown in Figs. 4 and 5, the absorbent body 1 is smaller than the backsheet 3, while the reinforcing member 14 and the liquid guide layer 15 are smaller than the absorbent body 1. These components are disposed such that all the centers of the reinforcing member 14, the absorbent body 1 and the liquid guide layer 15 coincide with the intersection of the transversely extending centerline Ox-Ox and the longitudinally extending centerline Oy-Oy on the backsheet 13.

As shown in Figs. 4 and 5, the sanitary napkin 11 is heat-embossed from the topsheet 16 toward the absorbent body 1 and the backsheet 13 to have recessed compressed portions 20. These recessed compressed portions 20 are preferably provided at locations which are spaced from the individual edges of the reinforcing member 14 and the liquid guide layer 15 so as not to overlap with the reinforcing member 14 and the liquid guide layer 15. That is, the recessed compressed portions 20 are preferably provided at locations where the topsheet 16, the absorbent body 1 and the backsheet 13 are stacked one on another.

The liquid guide layer 15 may be a spunlaced nonwoven fabric essentially consisting of rayon. For example, this spunlaced nonwoven fabric has a basis weight of 25 g/m² and is folded in three to have a length of 90 mm in the Y-direction and a width of 30 mm in the X-direction. The liquid guide layer 15 preferably has a basis weight in the range of 20 to 100 g/m². In the case where the liquid guide layer 15 of the spunlaced nonwoven fabric or the like is provided beneath the topsheet 16 at least centrally of the sanitary napkin 11, even if a shearing force acts on the topsheet 16 in a direction parallel to the plane of the body surface, such a shearing force can be relieved by the liquid guide layer 15. As a result, a shearing force acting between the tissue paper layer 3 and the absorbent core 2 can be decreased, so that separation of the tissue paper layer 3 from the absorbent core 2 at the first recesses 8' and the second recesses 8" can be prevented.

Body fluids such as menstrual blood given to the topsheet 16 in a region surrounded by the recessed compressed portions 20 pass through the topsheet 16, diffuse through the liquid guide layer 15, pass through the liquid guide layer 15, and are then introduced into the absorbent body 1. Thereafter, the body fluids diffuse through the tissue paper layer 3 of the absorbent body 1, and are then absorbed and retained by the underlying absorbent core 2. Here, the liquid guide layer 15 may be embossed to have a large number of dotted recesses aligned in the Y-direction or a large number of linear recesses extending in the Y-direction so that body fluids given to the liquid guide layer 15 can easily disperse in the Y-direction.

As has been described hereinabove, since the tissue paper layer 3 and the absorbent core 2 of the absorbent body 1 are in close contact with each other due to formation of the consolidated portions 7, body fluids given to the surface of the tissue paper layer 3 can rapidly pass through it to reach to the absorbent core 2.

Preferably, adjacent layers of the sanitary napkin 11 are bonded to each other through a hot-melt type adhesive. More specifically: with a hot-melt type adhesive, the topsheet 16 may be bonded to the liquid guide layer 15; the liquid guide layer 15 may be bonded to the tissue paper layer 3; in a region where the liquid guide layer 15 is not present, the topsheet 16 may be bonded to the tissue paper layer 3; the tissue paper layer 4 may be bonded to the reinforcing member 14; and in a region where the reinforcing member 14 is not present, the tissue paper layer 4 may be bonded to the backsheet 13. It should be noted that in the absorbent body 1, no adhesive is applied between the absorbent core 2 and the tissue paper layers 3, 4 that are joined together at the recesses 8. The hot-melt type adhesive can increase adhesion between the layers, but if they are adhered over an excessively large area, permeability and absorbency of liquid will be deteriorated. Therefore, the hot-melt type adhesive is preferably applied in a net, comb or spiral pattern. Since the topsheet 16, the liquid guide layer 15 and the tissue paper layer 3 are bonded to each other through the adhesive and the tissue paper layer 3 cannot be easily separated from the absorbent core 2, clearance is hardly caused between the tissue paper layer 3 and the absorbent core 2 even when an external force acts on the sanitary napkin 11 during wear. Therefore, body fluids having passed through the topsheet 16 and the liquid guide layer 15 can diffuse through the tissue paper layer 3, and can be immediately absorbed by the underlying absorbent core 2 that is in close contact with the tissue paper layer 3.

Next, the reinforcing member 14 will be described with reference to Figs. 6 and 7 . The reinforcing member 14 functions as a cushion layer and is also used to impart a predetermined stiffness to a liquid-absorbing region surrounded by the recessed compressed portions 20. As shown in Figs. 6 and 7, the reinforcing member 14 is provided at both body and garment surfaces with a plurality of circular protrusions 14a that are regularly arranged at a predetermined spacing. This prevents the reinforcing member 14 from having an excessively high stiffness. For example, the reinforcing member 14 is a laminate of a through-air bonded nonwoven fabric and spunbonded or meltblown nonwoven fabrics laid on the body and garment surfaces of the through-air bonded nonwoven fabric, which is compressed to have a low density in the protrusions 14a and a high density in the surroundings thereof.

In the absorbent body of the present invention, as has been described hereinabove, the tissue paper and the absorbent core can be firmly joined together so as not to separate from each other even in the case where heat-fusible fibers are not contained therein. As a result, the absorbent body of the present invention can easily withstand a shearing force acting on the tissue paper. Since the tissue paper can keep in contact with the absorbent core during use, migration of body fluid from the tissue paper to the absorbent core can be easily stabilized. In addition, the absorbent body of the present invention can be given an appropriate stiffness as a whole depending on the size, distribution ratio and distribution pattern of the recesses by embossing.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. An absorbent body comprising:
an absorbent core including cellulose fibers; and
hydrophilic paper covering a body surface and a garment surface of the absorbent core, wherein
the absorbent body is embossed in a predetermined dot pattern to have a plurality of recesses in which the absorbent core and the paper are compressed and integrated together.

2. An absorbent body as set forth in claim 1, wherein the paper is broken in at least some of the recesses.

3. An absorbent body as set forth in claim 1, wherein the recesses include first recesses and second recesses having a smaller area than the first recesses, and both the first and second recesses are dotted over the absorbent body.

4. An absorbent body as set forth in claim 3, wherein the first and second recesses are arranged such that each first recess is surrounded by a plurality of second recesses.

5. An absorbent body as set forth in claim 3, wherein the paper is broken in at least some of the second recesses.

6. An absorbent body as set forth in claim 1, wherein each recess has a maximum length of 0.5 to 4 mm at a bottom thereof, and a center-to-center distance between adjacent recesses is 2 to 8 mm.

7. An absorbent body as set forth in claim 1, wherein the absorbent body is compressed not only at the recesses but also as a whole.

8. An absorbent body as set forth in claim 1, which has a Gurley stiffness of 2.45 to 3.43 mN.

9. An absorbent article having the absorbent body of claim 1 disposed between a backsheet and a liquid permeable topsheet, wherein the paper covering the body surface and the garment surface of the absorbent core is bonded to both a member laid on a body surface of the absorbent body and a member laid on a garment surface of the absorbent body.
